# EUROPÄISCHE PATENTSCHRIFT

(11) **EP 3 706 823 B1**
(45) Veröffentlichungstag und Bekanntmachung des Hinweises auf die Patenterteilung: **14.04.2021**
(21) Anmeldenummer: 18807564.2
(22) Anmeldetag: 08.11.2018
(51) Int. Cl.: A61M 3/02

(54) **LAVAGE-SYSTEM**
IRRIGATION SYSTEM
SYSTÈME DE LAVAGE

(30) Priorität: 10.11.2017 DE 202017106855 U
(43) Veröffentlichungstag der Anmeldung: 16.09.2020
(73) Patentinhaber: UTK Solutions GmbH, 58507 Lüdenscheid (DE); Thiessies, Olaf, 58513 Lüdenscheid (DE)
(72) Erfinder: THIESSIES, Olaf, 58513 Lüdenscheid (DE)
(74) Vertreter: Patentanwälte Dörner & Kötter PartG mbB
(86) Internationale Anmeldenummer: PCT/EP2018/080622
(87) Internationale Veröffentlichungsnummer: WO 2019/092109

(56) Entgegenhaltungen:
- US-A- 5 882 319
- US-A1- 2013 144 211
- US-A1- 2015 182 685
- US-A1- 2017 119 939

## Beschreibung

Die Erfindung betrifft Lavage-System, umfassend ein Gehäuse, an dem ein Abzug, bewegbar montiert ist, wobei in dem Gehäuse ein Motor, ein Akkumulator, eine Pumpe und ein Getriebe angeordnet sind.

Lavage-Systeme dienen dazu, bei einer Operation am Knochen, wie zum Beispiel dem Einsetzen eines künstlichen Gelenks, Rückstände, die bei der Operation entstehen, auszuspülen, um Komplikationen nach der Operation zu vermeiden und die Heilung zu beschleunigen, zumal Rückstände die Haltbarkeit des künstlichen Gelenks beeinträchtigen und zu Infektionen im Bereich des Knochens führen können. Die Spülung soll dabei gründlich aber gleichzeitig schonend erfolgen.

Durch das Lavage-System wird ein pulsierender Strahl einer Flüssigkeit erzeugt. Die Flüssigkeit ist meist steriles Wasser oder eine wässrige Lösung, die auch pharmazeutisch wirksame Substanzen enthält. Das Spülwasser mit den Rückständen wird durch das Lavage-System über einen zweiten Kanal abgesaugt.

Lavage-Systeme umfassen eine Pumpe, die zum Erzeugen des pulsierenden Flüssigkeitsstrahls und zum Fördern der Flüssigkeit verwendet werden. Ein Antriebsmodul wird vor der Anwendung mit dem Lavage-System verbunden und dient dazu, die Pumpe anzutreiben.

Die bekannten Lavage-Systeme werden entweder durch Elektromotoren oder mittels Druckluftmotoren angetrieben. Nachteilig an den mit Druckluft betriebenen Lavage-Systemen ist, dass der Motor mit Druckluft oder einem komprimierten Gas versorgt werden muss. Zwar ist in Operationssälen üblicherweise eine Druckluftversorgung vorhanden; der Druckluftmotor muss aber entweder an eine Druckgasleitung angeschlossen sein, wodurch die Handhabung beeinträchtigt ist, oder einen Kompressor mit elektrischen Leitungen oder eine Druckgaspatrone umfassen, wodurch der Aufbau der Systeme aufwändig ist (vgl. bspw. DE 10 2011 018 708 A1).

Aus der US 2003/036 723 A1 ist ein Lavage-System bekannt, bei dem die Pumpe durch einen Elektromotor über ein Getriebe angetrieben wird. Die Energie für den Motor wird über ein Stromkabel zugeführt, wodurch die Handhabung ebenfalls eingeschränkt ist. Zur Vermeidung dieses Nachteils sind Batterie betriebene Lavage-Systeme bekannt (vgl. bspw. DE 696 02 175 T2).

Auch aus der US2015/182685 A1 ist ein batteriebetriebener Irrigator bekannt, bei dem eine Pumpe, ein Werkzeug und ein Antrieb an einem Gehäuse angeordnet sind. Aus der US 2013/144211 A1 ist ein Lavage-System bekannt, das einen Bohrantrieb, einen wieder aufladbaren Akkumulator und einen Elektromotor umfasst. Das System weist einen lösbaren Lavage-Aufsatz auf. Die US 5 882 319 A beschreibt ein Lavage-Gerät, bei dem in einem Gehäuse ein Elektromotor angeordnet ist, der von Batterien umgeben ist.

Zwar erfüllen die bekannten Lavage-Systeme ihre Aufgabe. Allerdings sind die Lavage-Systeme aufgrund der hygienischen Anforderungen üblicherweise als Einwegartikel konzipiert. Folglich werden nach der Benutzung die Systeme entsorgt, und zwar einschließlich aller Bestandteile, insbesondere des Antriebs und der elektrischen Energieversorgung. Das ist unter dem Aspekt der Schonung von Ressourcen sowie der Einsparung von Kosten nicht vertretbar.

Vor diesem Hintergrund liegt der Erfindung die Aufgabe zugrunde, ein Lavage-System zu schaffen, bei dem die Energieversorgung und die den Antrieb betreffenden Bestandteile wiederverwendbar sind. Gemäß der Erfindung wird diese Aufgabe durch ein Lavage-System mit den Merkmalen des Patentanspruchs 1 gelöst.

Mit der Erfindung ist ein Lavage-System geschaffen, bei dem die Energieversorgung und die den Antrieb betreffenden Bestandteile wiederverwendbar sind. Durch die Zusammenfassung von Motor und Akkumulator zu einer Antriebseinheit, welche auswechselbar ist, besteht die Möglichkeit, nach Benutzung des Lavage-Systems die Antriebseinheit aus dem Gehäuse zu entfernen und in das Gehäuse eines anderen Lavage-Systems einzusetzen. Die Antriebseinheit ist folglich für eine Vielzahl von Spülvorgängen in unterschiedlichen Gehäusen einsetzbar. Somit stellt das erfindungsgemäße Lavage-System eine ressourcenschonende und damit umweltschonende sowie kostengünstige Lösung bereit.

Die an der Antriebseinheit vorgesehene Handhabe ermöglicht einen leichten Aus- bzw. Einbau der Antriebseinheit in das Gehäuse. Mit Hilfe der an dem kopfseitigen Ende der Antriebseinheit vorgesehenen Anzeige kann der Benutzer über den Ladezustand des Akkumulators informiert werden.

In Weiterbildung der Erfindung ist die Antriebseinheit mit elektrischen Kontakten versehen. Die elektrischen Kontakte ermöglichen eine Verbindung zu den übrigen in dem Gehäuse verbauten Einzelteilen, insbesondere eine Kontaktierung mit dem Abzug, um bei Betätigung des Abzugs in der Antriebseinheit ein Signal auszulösen, welches den Motor aktiviert und somit einen Spülvorgang auslöst. Bevorzugt ist die Antriebseinheit spritzwassergeschützt oder flüssigkeitsdicht. Die spritzwassergeschützte oder flüssigkeitsdichte Ausgestaltung führt zum einen zu einer hohen Betriebssicherheit, zum anderen besteht dadurch die Möglichkeit, die Antriebseinheit bei Bedarf zu sterilisieren.

In vorteilhafter Ausgestaltung ragt aus der Antriebseinheit eine Welle, die an ihrem freien Ende mit einem Zahnrad versehen ist. Über die Welle mit dem Zahnrad erfolgt unter Zwischenschaltung eines weiteren Zahnrades der Antrieb der Pumpe. Hierdurch ist eine konstruktiv einfache Lösung hervorgerufen, die gleichzeitig eine hohe Betriebssicherheit sicherstellt.

In anderer Weiterbildung der Erfindung hat die Antriebseinheit ein kreisrundes Exzenterzahnrad, auf dem ein Exzenter angeordnet ist. Durch den Exzenter kann die rotierende Bewegung des Exzenterzahnrades in eine axiale Bewegung des Stößels der Pumpe umgewandelt werden, wodurch eine konstruktiv einfache und zugleich sehr robuste Art des Antriebs geschaffen ist.

Äußerst vorteilhaft ist der Akkumulator wieder aufladbar. Durch die Wiederaufladefähigkeit des Akkumulators besteht eine nahezu unbegrenzte Einsatzdauer, da nach Gebrauch die Antriebseinheit mit dem Akkumulator in eine Ladestation eingesetzt oder an eine solche angeschlossen werden kann, so dass die Entsorgung im Falle der vollständigen Entleerung des Akkumulators, wie sie von nicht ladbaren Akkumulatoren (Batterien) bekannt ist, vermieden ist.

Andere Weiterbildungen und Ausgestaltungen der Erfindung sind in den übrigen Unteransprüchen angegeben. Ausführungsbeispiele der Erfindung sind in den Zeichnungen dargestellt und werden nachfolgend im Einzelnen beschrieben. Es zeigen:
- Figur 1: die perspektivische Darstellung eines Lavage-Systems mit Einführhilfe und explosionsartig dargestellter Antriebseinheit;
- Figur 2: die perspektivische Darstellung des in Figur 1 dargestellten Lavage-Systems mit Einführhilfe und eingesetzter Antriebseinheit;
- Figur 3: die perspektivische Darstellung des in Figur 1 dargestellten Lavage-Systems mit entfernter Einführhilfe und geöffnetem Schiebedeckel;
- Figur 4: die perspektivische Darstellung des Lavage-Systems mit vollständig geöffneter Schiebeklappe;
- Figur 5: das in Figur 4 dargestellten Lavage-System mit teilweise geschlossener Schiebeklappe;
- Figur 6: das in Figur 4 dargestellte Lavage-System mit geschlossener Schiebeklappe;
- Figur 7: das in Figur 4 dargestellte Lavage-System mit explosionsartig dargestellter Antriebseinheit;
- Figur 8: die perspektivische Darstellung eines Lavage-Systems in anderer Ausbildung mit geöffnetem Deckel, Einführhilfe und Sprühdüse ohne Antriebseinheit;
- Figur 9: die perspektivische Darstellung des in Figur 8 dargestellten Lavage-Systems mit geöffnetem Deckel, Einführhilfe und Sprühdüse mit eingesetzter Antriebseinheit;
- Figur 10: die perspektivische Darstellung des in Figur 8 dargestellten Lavage-Systems mit teilweise aufgeschnitten dargestelltem Gehäuse und eingesetzter Antriebseinheit;
- Figur 11: die perspektivische Darstellung einer Antriebseinheit;
- Figur 12: die in Figur 11 dargestellte Antriebseinheit aus einer anderen Perspektive;
- Figur 13: die perspektivische Darstellung eines Klappdeckels;
- Figur 14: die perspektivische Darstellung eines Stößels.

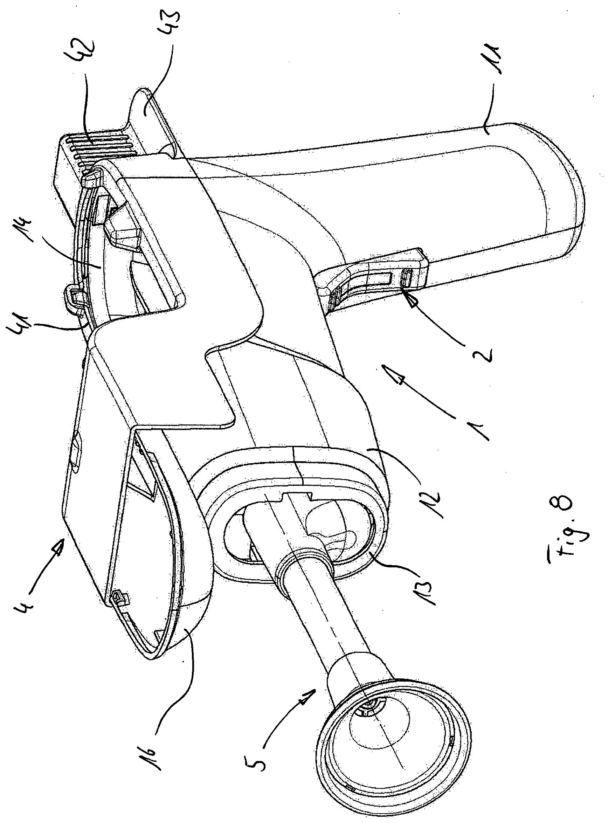

Das erfindungsgemäße Lavage-System umfasst ein Gehäuse 1 aus Kunststoff, welches aus einem Griffteil 11 und einem Funktionsteil 12 gebildet ist. An dem Gehäuse 1 ist ein Abzug 2 bewegbar montiert, wobei der Abzug 2 im Griffteil 11 angeordnet ist. Der Abzug 2 ist nach Art eines Kipphebels gebildet. An dem dem Griffteil 11 abgewandten Ende des Funktionsteils 12 ist eine Aufnahme 13 vorgesehen, die zum Anschluss von Aufsätzen dient. Die Aufsätze können in bekannter Weise gebildet sein und an ihrem freien Ende mit einem Spritzschutz versehen sein. Ein solcher Aufsatz ist in den Figuren 8 bis 9 dargestellt und mit "5" gekennzeichnet. Die Aufsätze sind in der Regel derart ausgebildet, dass zwei schlauchartige Leitungen vorgesehen sind, von denen die eine die Spüllösung in Richtung der zu spülenden Wunde fördert, die andere die Spüllösung mit den Verunreinigungen aus der Wunde absaugt. Die Aufsätze können zum Reinigen von Knochen, Weichgewebe oder zur Spülung ausgebildet sein. Auch können Bürstenaufsätze oder dergleichen Anwendung finden.

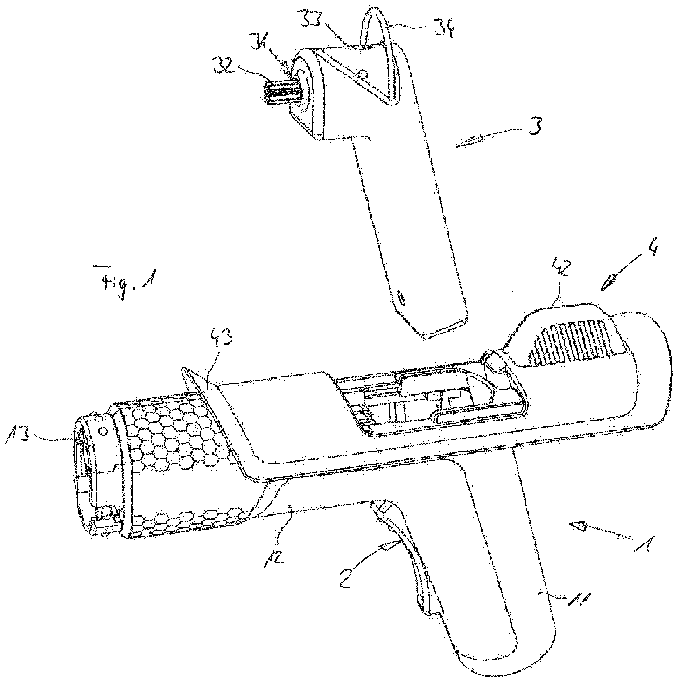

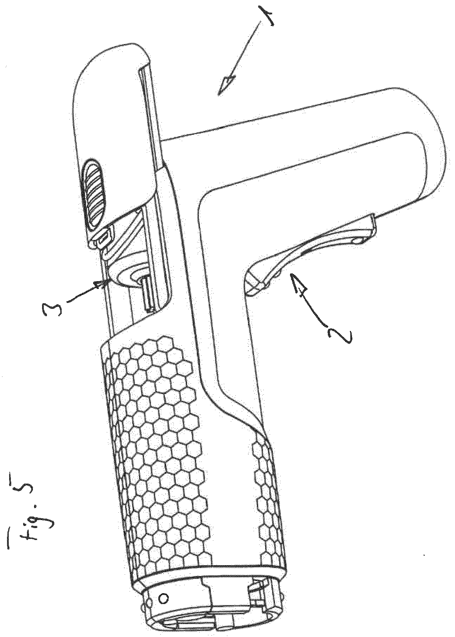

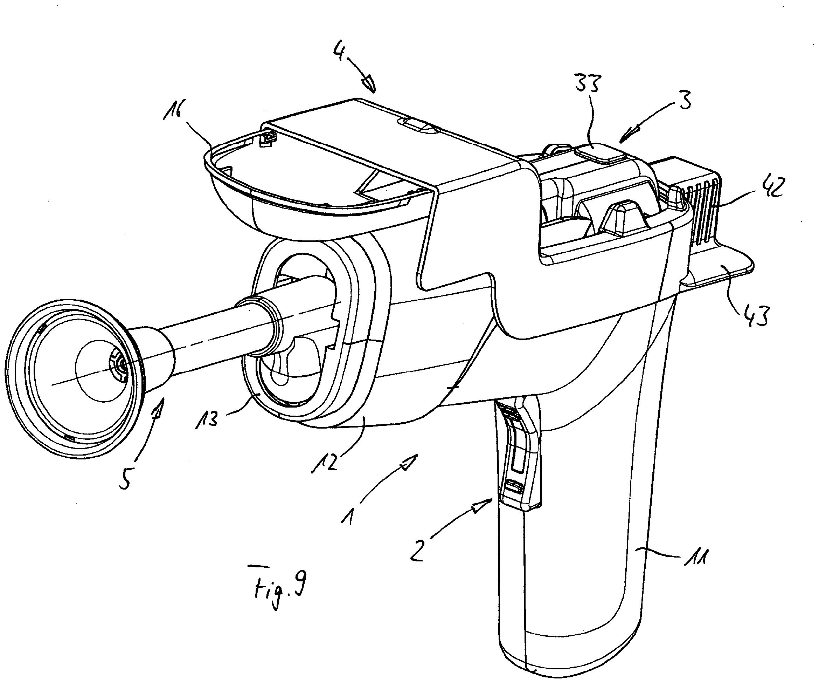

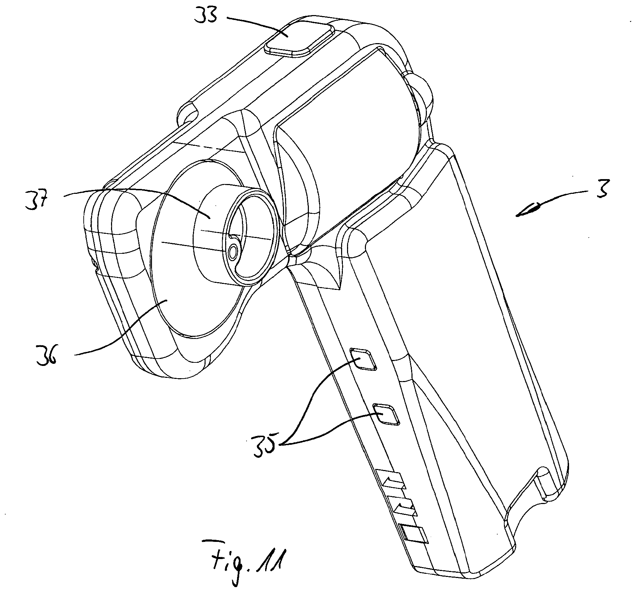

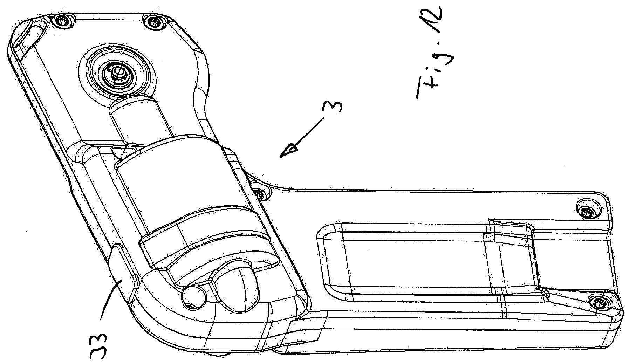

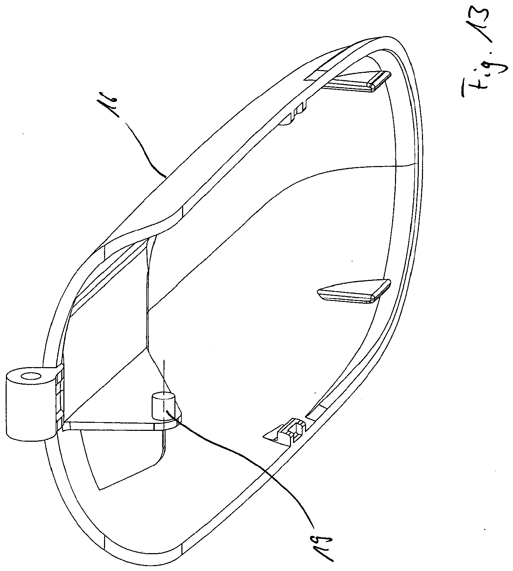

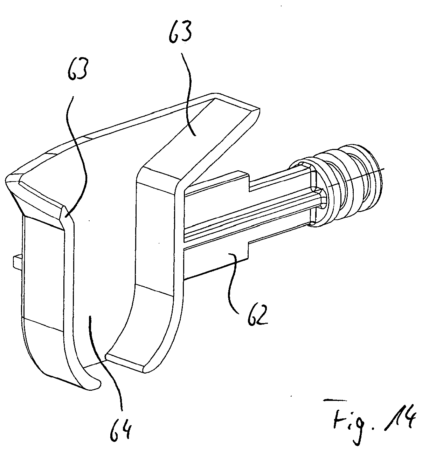

Das Gehäuse 1 ist mit einer Öffnung 14 versehen, die im Ausführungsbeispiel nach den Figuren 1 bis 7 mittels einer Schiebeklappe 15 verschließbar ist. Im Ausführungsbeispiel nach den Figuren 8 bis 14 ist die Öffnung dagegen mittels eines Klappdeckels 16 verschließbar, der um die Achse von an dem Gehäuse 1 vorgesehenen Scharnieren 17 schwenkbar ist (vgl. Figur 9). In verschlossenem Zustand ist der Klappdeckel 16 mittels an dem Gehäuse 1 vorgesehener Rastnasen 18 verriegelt. An dem Klappdeckel 16 ist zudem ein Niederhalter 19 ausgebildet

Vorzugsweise sind die Schiebeklappe 15 und der Klappdeckel 16 aus transparentem Material hergestellt. In dem Gehäuse 1 ist in dem Griffteil 11 ein Schacht ausgebildet, der eine Antriebseinheit 3 aufnimmt. In der Antriebseinheit 3 sind in den Ausführungsbeispielen ein Motor zum Betreiben des Lavage-Systems und ein Akkumulator verbaut. Der Akkumulator ist im Ausführungsbeispiel wiederaufladbar.

Die Antriebseinheit 3 ist mit elektrischen Kontakten 35 versehen, die mit elektrischen Kontakten in dem Gehäuse 1 korrespondieren. Auf diese Weise ist eine Verbindung zwischen der Antriebseinheit 3 und dem Abzug 2 geschaffen, so dass eine Betätigung des Abzugs 2 den Motor in der Antriebseinheit 3 startet. Gleichzeitig dienen die elektrischen Kontakte zum Aufladen des Akkumulators in der Antriebseinheit 3, indem die Antriebseinheit 3 in einer Ladestation positioniert wird oder an ein Ladegerät angeschlossen wird.

Die Antriebseinheit 3 ist flüssigkeitsdicht ausgebildet. An ihrem kopfseitigen Ende ragt aus der Antriebseinheit 3 im Ausführungsbeispiel nach den Figuren 1 bis 7 eine Welle 31, die an ihrem freien Ende mit einem Zahnrad 32 versehen ist. Das Zahnrad 32 ist vorzugsweise aus Hochleistungskunststoff hergestellt. Im Ausführungsbeispiel nach den Figuren 8 bis 14 hat die Antriebseinheit 3 dagegen seitlich ein kreisrundes Exzenterzahnrad 36, auf dem ein Exzenter 37 angeordnet ist. Die Exzenterscheibe 36 ist mit einem Freilauf versehen.

Darüber hinaus ist die Antriebseinheit 3 an ihrem kopfseitigen Ende mit einer An zeige 33 versehen, die den Ladestatus des Akkumulators anzeigt. Die Anzeige 33 ist unter einem Fenster angeordnet, um die spritzwassergeschützte bzw. flüssigkeitsdichte Ausgestaltung der Antriebseinheit 3 zu gewährleisten. Die Antriebseinheit 3 ist zudem mit einer Handhabe 34 versehen, die im Ausführungsbeispiel nach den Figuren 1 bis 7 aus einem schwenkbaren Bügel besteht. Der schwenkbare Bügel hat eine Form, die im Wesentlichen der Form des kopfseitigen Endes der Antriebseinheit 3 entspricht. In nicht benutztem Zustand kann die Handhabe 34 daher derart eingeklappt werden, dass sie sich an die Kontur der Antriebseinheit 3 anlegt.

In der Antriebseinheit 3 sind auf einer Platine Wärmesensoren vorgesehen, die die Betriebstemperatur der Antriebseinheit 3 insbesondere im Bereich des Motors überwachen. Sie stehen mit der Anzeige 33 in Verbindung. Sollten die Sensoren eine zu hohe Betriebstemperatur messen, wird ein Signal an die Anzeige 33 gesandt. Die Anzeige 33 signalisiert daraufhin dem Benutzer den fehlerhaften Betriebszustand. Auf der Platine können auch eine Stromüberwachung und eine Ladeelektronik angeordnet sein.

Die Antriebseinheit 3 ist mittels einer sog. Push-Push-Verriegelung in dem Gehäuse 1 arretierbar. Das bedeutet, dass die Antriebseinheit 3 beim Einsetzen in das Gehäuse 1 von oben durch die Öffnung 14 bei Erreichen ihrer Funktionsstellung einrastet. In geschlossenem Zustand des Klappdeckels 16 hält dabei der Niederhalter 19 die Antriebseinheit 3 in ihrer Funktionsstellung. Zum Entriegeln der Antriebseinheit 3 ist nach Öffnen der Schiebeklappe 15 bzw. des Klappdeckels 16 lediglich das Ausüben eines Drucks auf die Antriebseinheit 3 von oben erforderlich, wodurch die Antriebseinheit 3 aus ihrer verriegelten Position befreit wird.

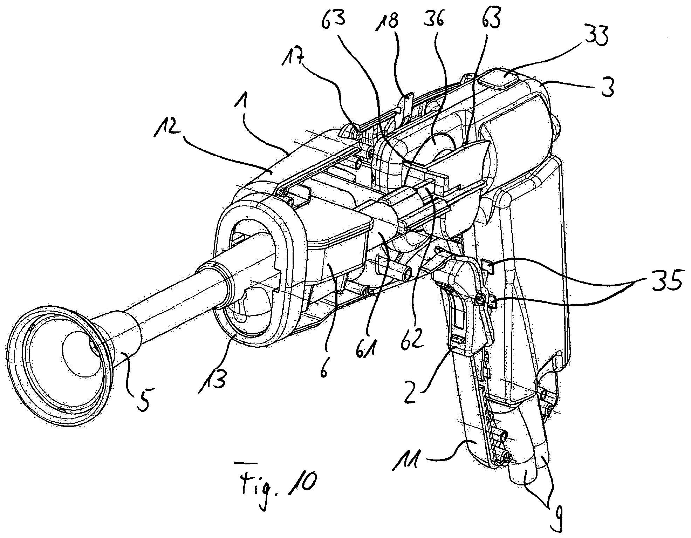

In dem Gehäuse 1 ist eine Pumpe 6 angeordnet. Die Pumpe 6 ist vorzugsweise zweiteilig ausgebildet und mit einem Stößel 62 versehen, der in einer Führung 61 geführt ist. Die Pumpe 6 wirkt zusammen mit einem ebenfalls innerhalb des Gehäuses angeordneten Getriebe, wobei als Getriebe die Verbindung der Pumpe 6 mit der Antriebseinheit 3 bezeichnet. Das Getriebe steht wiederum in Kontakt mit dem Zahnrad 32 gemäß Ausführungsbeispiel nach den Figuren 1 bis 7. Im Ausführungsbeispiel nach den Figuren 8 bis 14 ist das Getriebe gebildet von dem Exzenter 37 und einer Aufnahme 64, in der in montiertem Zustand der Exzenter 37 positioniert ist. Bei der Pumpe 6 kann es sich um eine Vakuumpumpe, Membranpumpe oder dergleichen handeln. Die Pumpe 6 ist angeschlossen an zwei Schläuche 9, die in dem Gehäuse 1 vorgesehen sind und die an dem dem Funktionsteil 22 abgewandten Ende aus den Griffteil 21 austreten (vgl. Figur 10). Die Schläuche 9 stehen einerseits in Verbindung mit einem Vorratsbehälter für die Spülflüssigkeit, andererseits mit einem Auffangbehälter für die aus der Wunde abgesaugte Flüssigkeit. Die Schläuche 9 verlaufen innerhalb des Gehäuses 1 unter einer - nicht dargestellten - Abdeckung, um einen Kontakt mit der Antriebseinheit 3 zu vermeiden.

Das erfindungsgemäße Lavage-System wird mit einer Einführhilfe 4 ausgeliefert. Die Einführhilfe 4 ist auf das Gehäuse 1 geklemmt und abnehmbar. Wie den Figuren 1 und 2 sowie 8 und 9 zu entnehmen ist, überdeckt die Einführhilfe 4 in montiertem Zustand die Oberseite des Gehäuses 1. Sie weist eine Aussparung 41 auf, deren Größe und Form im Wesentlichen derjenigen der Öffnung 14 im Gehäuse 1 entspricht. Benachbart zur Aussparung 41 ist ein Griff 42 ausgebildet. Zudem hat die Einführhilfe 4 einen Rand 43, der im Ausführungsbeispiel nach den Figuren 1 bis 7 umlaufend gestaltet ist.

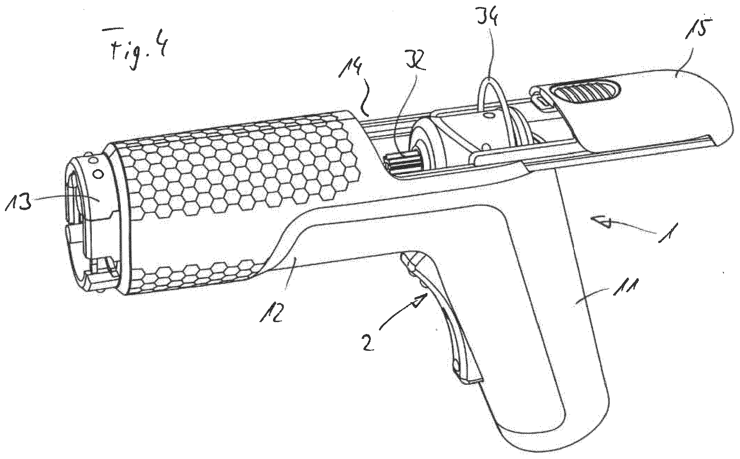

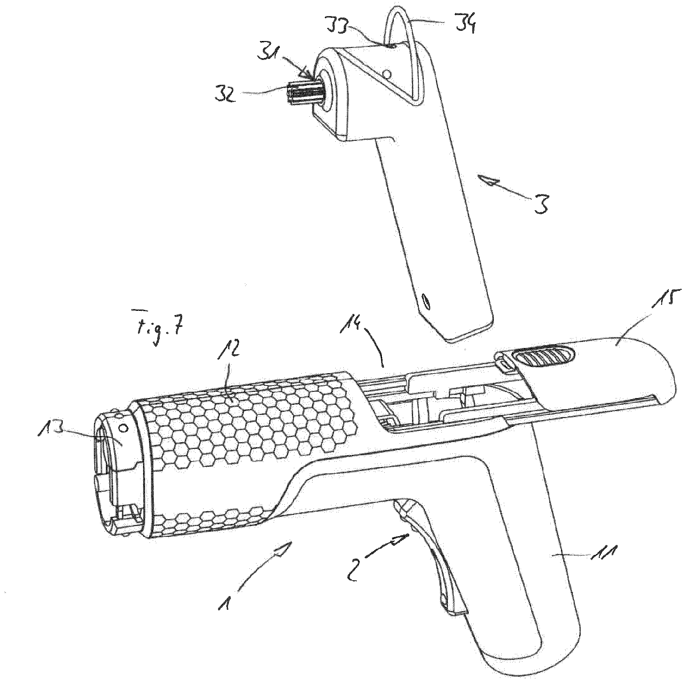

Bei dem erfindungsgemäßen Lavage-System sind der Motor und der Akkumulator in der Antriebseinheit 3 verbaut. Die Antriebseinheit 3 ist auswechselbar, wie insbesondere Figur 1 und 7 zu entnehmen ist. Zum Einsetzen der Antriebseinheit 3 ist die Schiebeklappe 25 in die der Aufnahme 23 abgewandte Richtung verschoben, wie dies in Figur 4 dargestellt ist; im Ausführungsbeispiel nach Figur 9 ist der Klappdeckel 16 aufgeklappt. Gleichzeitig ist die Einführhilfe 4 an dem Gehäuse 1 angebracht. Wie bspw. in Figur 1 dargestellt, kann der Bediener die Antriebseinheit 3 von oben in das Gehäuse 1 einsetzen, in dem er die Antriebseinheit 3 durch die Aussparung 41 und die Öffnung 14 entlang des in dem Griffteil 11 ausgebildeten Schacht einführt. Beim Einführen kommt im Ausführungsbeispiel nach den Figuren 8 bis 13 der Exzenter 37 mit Einlaufschrägen 63 in Kontakt, die aufgrund des Freilaufs eine Drehung des Exzenterzahnrades 36 zur Folge hat und den Exzenter 37 in eine konstruktiv vorbestimmte Position in der Aufnahme 64 bringen. Dadurch nimmt der Exzenter 37 in der Funktionsstellung der Antriebseinheit 3 automatisch die zum Betreiben der Pumpe 6 notwendige Position ein.

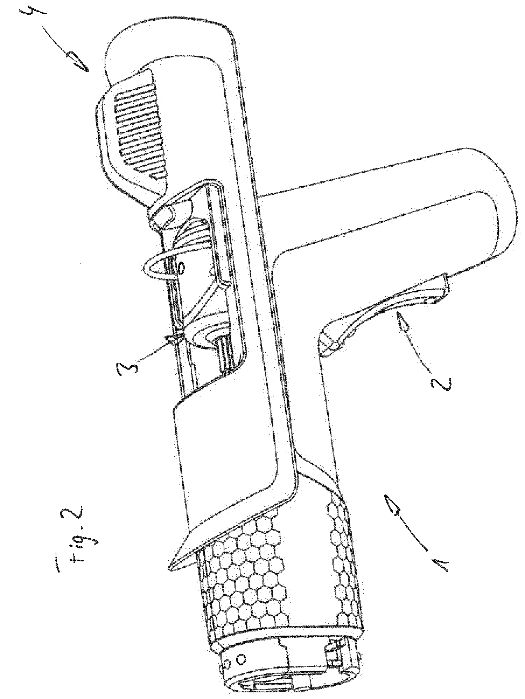

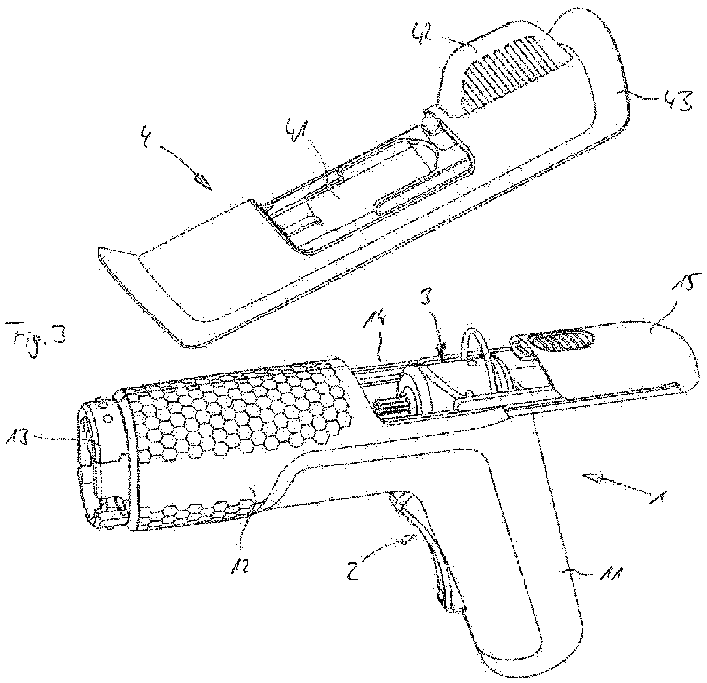

Wenn die Antriebseinheit 3 in das Gehäuse 1 eingeführt ist, nimmt sie die in den Figuren 2 und 9 dargestellte Position ein. Die Einführhilfe 4 wird dann vom Gehäuse 1 demontiert (Figur 3). In dieser eingenommenen Arbeitsposition treten die Kontakte an der Antriebseinheit 3 mit den Kontakten im Gehäuse 1 in Berührung. Gleichzeitig befindet sich in dieser Position das Zahnrad 32 bzw. der Exzenter 37 in der zum Betreiben der Pumpe 6 geeigneten Stellung. Mit Hilfe des auf des beim Betrieb rotierenden Exzenterzahnrades 36 angeordneten Exzenters 37 wird die Rotationsbewegung des Antriebs in eine axiale Bewegung des Stößels der Pumpe 6 umgewandelt.

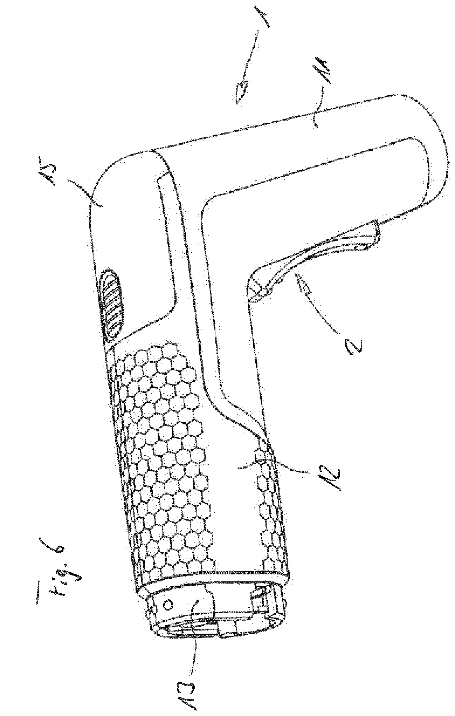

Nach Einklappen der Handhabe 34 wird im Ausführungsbeispiel nach den Figuren 1 bis 7 die Schiebeklappe 15 in Richtung der Aufnahme 13 geschoben, bis sie ihre Schließposition erreicht hat, wie dies in Figur 6 dargestellt ist. Im Ausführungsbeispiel nach den Figuren 8 bis 14 wird der Klappdeckel 16 um die von den Scharnieren 17 gebildete Achse geklappt bis der Deckel mit den Rastnasen 18 verrastet. Da die Schiebeklappe 15 bzw. der Klappdeckel 16 transparent ausgeführt ist, ist die Anzeige 33 der Antriebseinheit 3 auch bei geschlossener Schiebeklappe 15 bzw. geschlossenem Klappdeckel 16 sichtbar. Nach Anschließendes Lavage-Systems an die Flüssigkeits- bzw. Auffangbehälter und Anordnung der jeweils gewünschten Aufsätze an der Aufnahme 13 kann durch Betätigen des Abzugs 2 das Lavage-System in Betrieb genommen werden. Dies erfolgt in an sich aus anderen Lavage-Systemen bekannter Weise.

Nach der Benutzung des erfindungsgemäßen Lavage-Systems kann durch Öffnen der Schiebeklappe 15 bzw. des Klappdeckels 16 die Antriebseinheit 3 dem Gehäuse 1 entnommen werden. Hierzu wird zunächst von oben auf die Antriebseinheit 3 gedrückt, um diese zu entriegeln. Sodann kann die Handhabe 34 nach oben geklappt werden, so dass der Benutzer diese ergreifen und die Antriebseinheit 3 aus dem Gehäuse 1 entnehmen kann. Nach der Entnahme der Antriebseinheit 3, wie es bspw. in Figur 7 dargestellt ist, kann das nur aus Kunststoff bestehende Gehäuse 1 mit den darin angeordneten Komponenten entsorgt werden. Die Antriebseinheit 3 selbst kann einer Ladestation zugeführt werden, um den wiederaufladbaren Akkumulator mit Strom zu speisen.

Aufgrund der spritzwassergeschützten oder flüssigkeitsdichten Ausgestaltung der Antriebseinheit 3 besteht grundsätzlich die Möglichkeit, die Antriebseinheit 3 mit den darin angeordneten Komponenten zu reinigen und/oder zu sterilisieren. Infolgedessen sind alle Anforderungen an die Hygiene im Operationsbereich erfüllt. Es bestehen dabei keine hygienischen Bedenken, da es beim Einsetzen der Antriebseinheit 3 lediglich zu einem Kontakt mit der Einführhilfe 4 kommen kann, die aber nach dem Einführen entfernt wird. Folglich bleibt das Gehäuse 1 außen auch bei Einsetzen einer nicht sterilen Antriebseinheit 3 steril.

Durch die mannigfache Verwendbarkeit der Antriebseinheit 3 ist die aus dem Stand der Technik bekannte Entsorgung vollständiger Lavage-Systeme einschließlich Antrieb und Energieversorgung vermieden, wodurch ein erheblicher Beitrag zum Umweltschutz und zur Reduzierung der Kosten geleistet ist. Aufgrund des ausschließlich aus Kunststoff bestehenden Gehäuses 1 einschließlich Abzug 2, Pumpe 6 und Getriebe besteht zudem die Möglichkeit eines sehr einfachen Recyclings, da auf die vorherige Trennung einzelner Komponenten aus unterschiedlichen Materialien, insbesondere von Metallen, verzichtet werden kann. Zudem ist aufgrund der für das Gehäuse 1 gewählten Kunststoffe eine sichere und zugleich ermüdungsfreie Handhabung ermöglicht. Außerdem ist das erfindungsgemäße Lavage-System handhabungsfreundlich aufgrund seiner ergonomischen Gestaltung und bietet somit dem Operateur eine bequeme und ermüdungsfreie Bedienung.

## Patentansprüche

1. Lavage-System, umfassend ein Gehäuse (1), an dem ein Abzug (2) bewegbar montiert ist, wobei in dem Gehäuse (1) ein Motor, ein Akkumulator, eine Pumpe (6) und ein Getriebe angeordnet sind, wobei wenigstens der Motor und der Akkumulator in einer Antriebseinheit (3) verbaut sind, die auswechselbar ist , **dadurch gekennzeichnet, dass** die Antriebseinheit (3) mit einer Handhabe (34) versehen ist und von oben durch eine Öffnung (14) in das Gehäuse (1) einsetzbar ist, wobei die Öffnung mit einer Schiebeklappe (15) oder einem Klappdeckel (16) verschließbar ist, die aus transparentem Kunststoff bestehen, durch die in geschlossenem Zustand eine am kopfseitigen Ende der Antriebseinheit (3) vorgesehene Anzeige (33) sichtbar ist.

2. Lavage-System nach Anspruch 1, **dadurch gekennzeichnet, dass** die Antriebseinheit (3) mit elektrischen Kontakten versehen ist.

3. Lavage-System nach Anspruch 1 oder 2, **dadurch gekennzeichnet, dass** die Antriebseinheit (3) spritzwassergeschützt ist.

4. Lavage-System nach Anspruch 1 oder 2, **dadurch gekennzeichnet, dass** die Antriebseinheit (3) flüssigkeitsdicht ist.

5. Lavage-System nach einem der vorgenannten Ansprüche, **dadurch gekennzeichnet, dass** aus der Antriebseinheit (3) eine Welle (31) ragt, die an ihrem freien Ende mit einem Zahnrad (32) versehen ist.

6. Lavage-System nach einem der vorgenannten Ansprüche, **dadurch gekennzeichnet, dass** die Antriebseinheit (3) ein kreisrundes Exzenterzahnrad (36) hat, auf dem ein Exzenter (37) ausgebildet ist.

7. Lavage-System nach einem der vorgenannten Ansprüche, **dadurch gekennzeichnet, dass** der Akkumulator wieder aufladbar ist.

8. Lavage-System nach einem der vorgenannten Ansprüche, **dadurch gekennzeichnet, dass** eine Einführhilfe (4) vorgesehen ist.

9. Lavage-System nach einem der vorgenannten Ansprüche, **dadurch gekennzeichnet, dass** das Gehäuses (1), Abzug (2), Pumpe (6) und Getriebe aus Kunststoff bestehen.

## Claims

1. Irrigation system comprising a housing (1) on which a trigger (2) is movably mounted, wherein a motor, an accumulator, a pump (6) and a gear mechanism are arranged in the housing (1), wherein at least the motor and the accumulator are built into a drive unit (3) which is replaceable, **characterised in that** the drive unit (3) is provided with a handle (34) and can be inserted in the housing (1) from above through an opening (14), wherein the opening can be closed by a sliding flap (15) or a flap cover (16) which consists of clear plastic, through which a display (33) provided at the head-side end of the drive unit (3) is visible in the closed state.

2. Irrigation system according to claim 1, **characterised in that** the drive unit (3) is provided with electrical contacts.

3. Irrigation system according to claim 1 or 2, **characterised in that** the drive unit (3) is splash-proof.

4. Irrigation system according to claim 1 or 2, **characterised in that** the drive unit (3) is liquid-tight.

5. Irrigation system according to one of the previous claims, **characterised in that** a shaft (31) protrudes from the drive unit (3), which shaft is provided with a gear wheel (32) at its free end.

6. Irrigation system according to one of the previous claims, **characterised in that** the drive unit (3) has a circular eccentric gear wheel (36), on which an eccentric is formed.

7. Irrigation system according to one of the previous claims, **characterised in that** the accumulator is rechargeable.

8. Irrigation system according to one of the previous claims, **characterised in that** an insertion aid (4) is provided.

9. Irrigation system according to one of the previous claims, **characterised in that** the housing (1), trigger (2), pump (6) and gear mechanism are made of plastic.

## Revendications

1. Système de lavage, comprenant un boîtier (1) contre lequel une gâchette (2) est montée mobile, sachant que dans le boîtier (1) sont disposés un moteur, un accumulateur, une pompe (6) et une transmission, sachant qu'au moins le moteur et l'accumulateur sont montés dans une unité d'entraînement (3) interchangeable, **caractérisé en ce que** l'unité d'entraînement (3) est munie d'un étrier (34) et qu'elle peut être introduite par le haut dans le boîtier (1) via un orifice (14), sachant que l'orifice est obturable à l'aide d'un capot coulissant (15) ou d'un couvercle rabattable (16) composés de matière plastique transparente à travers laquelle est visible, à l'état fermé, un affichage (33) prévu à l'extrémité têtière de l'unité d'entraînement (3).

2. Système de lavage selon la revendication 1, **caractérisé en ce que** l'unité d'entraînement (3) est munie de contacts électriques.

3. Système de lavage selon la revendication 1 ou 2, **caractérisé en ce que** l'unité d'entraînement (3) est protégée contre les projections d'eau.

4. Système de lavage selon la revendication 1 ou 2, **caractérisé en ce que** l'unité d'entraînement (3) est étanche aux liquides.

5. Système de lavage selon l'une des revendications précédentes, **caractérisé en ce que** de l'unité d'entraînement (3) fait saillie un arbre (31) qui sur son extrémité libre est muni d'un pignon (32).

6. Système de lavage selon l'une des revendications précédentes, **caractérisé en ce que** l'unité d'entraînement (3) présente un pignon circulaire (36) à excentrique sur lequel est configuré un excentrique (37).

7. Système de lavage selon l'une des revendications précédentes, **caractérisé en ce que** l'accumulateur est rechargeable.

8. Système de lavage selon l'une des revendications précédentes, **caractérisé en ce qu'**une aide à l'introduction (4) est prévue.

9. Système de lavage selon l'une des revendications précédentes, **caractérisé en ce que** le boîtier (1), la gâchette (2), la pompe (6) et la transmission sont en matière plastique.
